# EUROPEAN PATENT APPLICATION

(11) **EP 3 088 047 A1**
(43) Date of publication of application: **02.11.2016**
(21) Application number: 14878740.1
(22) Date of filing: 16.01.2014
(51) Int. Cl.: A61N 5/10

(54) **LEAF POSITION DETECTION DEVICE, MULTI-LEAF COLLIMATOR, AND RADIATION THERAPY DEVICE**

(71) Applicant: Mitsubishi Heavy Industries, Ltd., Tokyo 108-8215 (JP)
(72) Inventor: ARAI, Satoshi, Tokyo 108-8215 (JP)
(74) Representative: Hoffmann Eitle
(86) International application number: PCT/JP2014/050680
(87) International publication number: WO 2015/107651

(57) **Abstract**

A mark position detection unit (200) includes an illumination unit (210) which illuminates an identification mark (102) provided on a leaf (70) with detection light (L1), and a detection unit (220) which detects a light (L2) emitted from the identification mark (102) which is illuminated with the detection light (L1). The illumination unit (210) includes a light source (211) which emits the detection light (L1). The detection unit (220) includes a polarizing filter (221) which filters the light (L2) emitted from the identification mark (102) and an imaging device (222) which detects a transmitted light filtered by the polarizing filter (221).

## Description

### [Technical Field]

The present invention relates to a leaf position detection device of a multi-leaf collimator which limits a radiation field of radiation radiated for performing radiation therapy, the multi-leaf collimator, and a radiation therapy device.

### [Background Art]

Radiation therapy in which an affected body area is irradiated with radiation is one way of treating tumors. In radiation therapy, the affected body area needs to be efficiently irradiated with radiation while an amount (dose) of radiation with which the patient is irradiated is kept as low as possible.

Consequently, a multi-leaf collimator to limit a radiation field, which is an area or a shape in which radiation is radiated, is being used in radiation irradiation devices.

A plurality of leaves in the shape of thin plates are disposed in parallel within a frame at predetermined intervals in the thickness direction of each plate, in the multi-leaf collimator. In addition, each of the leaves can be moved by a driving mechanism and is individually advanced and retracted within an irradiation range. When the leaves are disposed in the irradiation range, radiation is shielded by the leaves and the radiation field is limited. That is, a radiation field appropriate for each patient can be formed by limiting the radiation field in this way.

There are many cases in which the driving mechanism mentioned above includes a servomotor. In this case, the driving mechanism controls movement amounts of the leaves by detecting a rotational amount of the servomotor using an encoder. To further improve precision of the positions of the leaves in the irradiation range, for example, results of actual positions of leaves detected is provided as feedback for controlling the positions of the leaves in the driving mechanism.

For example, in Patent Literature 1, a technology in which image of a marker provided on each leaf is captured with a camera and position of the marker is identified by processing the captured image to detect positions of the leaves is disclosed.

In the technology according to Patent Literature 1, there are cases in which a marker is disposed at a place to which an outside light does not reach into a radiation irradiation device. Due to this issue, in Patent Literature 1, an illumination device is provided and image of a marker illuminated with illumination light from the illumination device is captured with a camera.

### [Citation List]

### [Patent Literature]

### [Patent Literature 1]

Japanese Patent Publication No. 4436342

### [Summary of Invention]

### [Technical Problem]

However, the light obtained from the marker described above is generally reflected light. For example, in the case in which a fluorescent substance is used in the marker, when the marker is illuminated with illumination light, visible light with a different wavelength from that of the illumination light is excited. The intensity of light reflected or excited from the marker becomes higher as the wavelength of the illumination light becomes shorter. Thus, when ultraviolet rays are used as the illumination light in the case in which a fluorescent substance is used in the marker, reflected light with a high intensity is obtained from the fluorescent substance such that the marker can easily be identified from an image captured with a camera. However, the cost of the illumination device increases when ultraviolet rays are used as the illumination light. In addition, a structure which prevents the ultraviolet rays from leaking to the outside is required.

On the other hand, when visible light with a wavelength longer than that of the illumination light is used, reflected light or excited light is weakened. Due to this issue, there are cases in which it becomes difficult to identify a marker from an image captured with a camera. For example, light reflected or excited from the marker may be intensified by increasing an amount of the illumination light. However, there is a possibility of causing so-called halation in which the whole image captured with a camera is whitened and blurred due to intense light which is reflected or excited.

The present invention has been devised in consideration of the circumstances above and is directed to providing a leaf position detection device capable of accurately identifying a marker provided on a leaf, a multi-leaf collimator, and a radiation therapy device.

### [Solution to Problem]

The present invention employs the following means to solve the problem above.

According to a first aspect of the present invention, a leaf position detection device is a leaf position detection device which detects a position of a leaf of a multi-leaf collimator having a plurality of the leaves which limit a radiation field, and includes an illumination unit which illuminates an identification mark provided on the leaf with detection light and a detection unit which detects a light emitted from the identification mark illuminated with the detection light, wherein the illumination unit includes a light source which emits the detection light, and the detection unit includes a polarizing filter which filters the light emitted from the identification mark and an imaging device which detects a transmitted light filtered by the polarizing filter.

According to a second aspect of the present invention, in the leaf position detection device, the identification mark on the leaf position detection device according to the first aspect may be composed of a fluorescent substance.

According to a third aspect of the present invention, in the leaf position detection device, the illumination unit in the leaf position detection device according to the first or second aspect may further include a polarizing plate which polarizes the detection light radiated from the light source.

According to a fourth aspect of the present invention, a multi-leaf collimator is a multi-leaf collimator which limits a radiation range, and includes a plurality of leaves arranged in their thickness direction, a driving mechanism which advances and retracts each of the leaves with respect to the radiation, an identification mark provided on each of the leaves and identifiable at least from a direction perpendicular to both the advancing and retracting direction in which the leaf is advanced and retracted and the thickness direction of the leaf, and the leaf position detection device according to any one aspect of the first to third aspects.

According to a fifth aspect of the present invention, a radiation therapy device includes the multi-leaf collimator according to the fourth aspect, a control unit which controls the driving mechanism based on a position of the identification mark detected by the leaf position detection device, and a radiation irradiation device which radiates radiation.

### [Advantageous Effects of Invention]

According to a leaf position detection device, a multi-leaf collimator, and a radiation therapy device of the present invention, identification of an identification mark provided on a leaf can be ensured.

### [Brief Description of Drawings]

Fig. 1 is a view illustrating a functional constitution of a radiation therapy system according to an embodiment of the present invention.
Fig. 2 is a perspective view illustrating a schematic constitution of a radiation therapy device which constitutes the radiation therapy system.
Fig. 3 is a cross-sectional view illustrating a radiation irradiation device which constitutes the radiation therapy device.
Fig. 4 is a perspective view illustrating an exterior of a multi-leaf collimator which constitutes a part of the radiation irradiation device.
Fig. 5 is a cross-sectional view of the multi-leaf collimator in a width direction.
Fig. 6 is a cross-sectional view of the multi-leaf collimator in a direction perpendicular to a plate thickness direction of a leaf.
Fig. 7 is a perspective view illustrating a leaf and a driving device which drives the leaf.
Fig. 8 is a perspective view illustrating a constitution of the driving device.
Fig. 9 is a view illustrating a schematic constitution of a leaf position detection device for detecting a position of the leaf.
Fig. 10 is a view illustrating a constitution of a leaf position detection device according to a first modified example of the embodiment.
Fig. 11 is a view illustrating a constitution of a leaf position detection device according to a second modified example of the embodiment.

### [Description of Embodiments]

Hereinafter, a leaf position detection device, a multi-leaf collimator, and a radiation therapy device according to an embodiment of the present invention will be described with reference to the drawings.

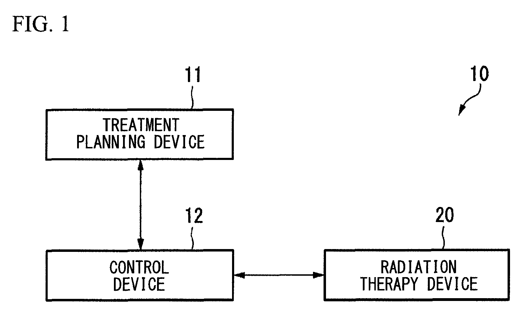

Fig. 1 is a view illustrating a functional constitution of a radiation therapy system 10 according to an embodiment of the present invention.

As illustrated in Fig. 1, the radiation therapy system 10 includes a treatment planning device 11, a control device (control unit) 12, and a radiation therapy device 20.

The treatment planning device 11 is a device in which properties of radiation with which a patient will be irradiated (intensity, time, angle, position, irradiation area, etc. of radiation with which the patient will be irradiated) are preset according to details of radiation therapy carried out on the patient are input from the outside. The treatment planning device 11 outputs values of various types of parameters for controlling radiation to be radiated according to the properties of radiation input to the control device 12.

The control device 12 controls an operation of the radiation therapy device 20 based on the values of the various types of parameters generated by the treatment planning device 11. The control device 12 is a computer device such as a personal computer which executes processing on the basis of a predetermined program. The control device 12 is connected to the radiation therapy device 20 via a wireless or wired communication line so as to be capable of two-way transmission of information.

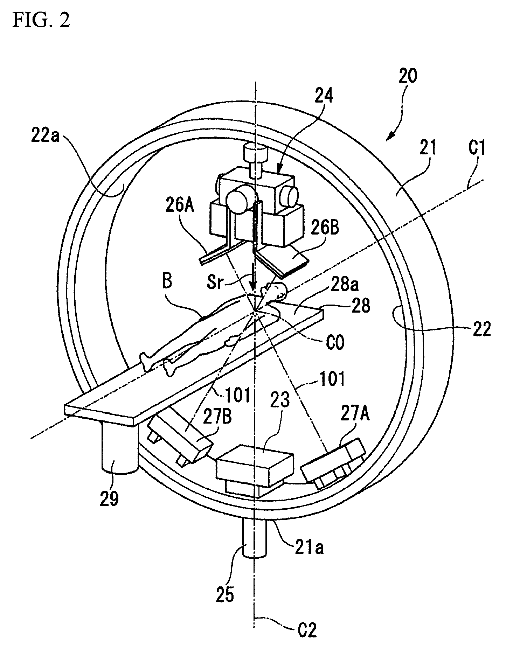

Fig. 2 is a perspective view illustrating a schematic constitution of the radiation therapy device 20 which constitutes the radiation therapy system 10.

As illustrated in Fig. 2, the radiation therapy device 20 includes a ring frame 21, a travelling gantry 22, and a radiation irradiation device 24.

The ring frame 21 is formed in a cylindrical shape having a circular cross-section. The ring frame 21 is disposed so that a central axis C1 substantially directs a horizontal direction. The ring frame 21 includes a rotation shaft 25 which extends downward integrally formed with an outer circumferential surface of a lower end portion 21a thereof. The rotation shaft 25 is supported by a base (not illustrated) in a state in which it is capable of rotating about a central axis C2 thereof. The rotation shaft 25 is driven to rotate by a rotation driving mechanism (not illustrated). That is, the ring frame 21 rotates about a perpendicular axis by the rotation shaft 25 rotated by the rotation driving mechanism.

The travelling gantry 22 is formed in a cylindrical shape having a circular cross-section. The travelling gantry 22 is disposed at an inner circumferential side of the ring frame 21. The travelling gantry 22 is supported by the ring frame 21 and is rotatable along an inner circumferential surface of the ring frame 21. In other words, the ring-shaped travelling gantry 22 is capable of rotating about the central axis C1 which extends in the horizontal direction. The travelling gantry 22 is rotated in the circumferential direction by a gantry driving mechanism (not illustrated).

The radiation irradiation device 24 is controlled by the control device 12 (refer to Fig. 1) and radiates therapeutic radiation Sr. The radiation irradiation device 24 is supported by an inner circumferential surface 22a of the travelling gantry 22. The therapeutic radiation Sr radiated from the radiation irradiation device 24 is adjusted to pass through an isocenter C0 which is an intersection point between the central axis C2 of the rotational movement of the ring frame 21 and the central axis C1 of the rotational movement of the travelling gantry 22.

As the radiation irradiation device 24 is supported by the travelling gantry 22 in this manner, the therapeutic radiation Sr is radiated to always pass through the isocenter C0 regardless of the rotational movement of the ring frame 21 about the central axis C2 and the rotational movement of the travelling gantry 22 about the central axis C1.

The radiation therapy device 20 further includes a sensor array 23. The sensor array 23 receives the therapeutic radiation Sr that has been radiated by the radiation irradiation device 24 and transmitted through a subject near the isocenter C0 and generates a transmission image of the subject. A flat panel detector (FPD), an X-ray image intensifier (II), etc. may be used as the sensor array 23.

In addition, the radiation therapy device 20 includes diagnostic X-ray sources 26A and 26B and sensor arrays 27A and 27B.

The diagnostic X-ray sources 26A and 26B are disposed at an inner circumferential side of the travelling gantry 22. The diagnostic X-ray sources 26A and 26B are disposed at both sides of the center of the radiation therapy device 20 (in other words, the central axis C2 of the rotational movement of the ring frame 21), along to the circumferential direction of the ring frame 21 interposed therebetween. The diagnostic X-ray sources 26A and 26B are controlled by the control device 12 and radiate diagnostic X-rays 101 toward the isocenter C0. The diagnostic X-rays 101 are a conical beam diffused in a conical shape from one point of the diagnostic X-ray sources 26A and 26B.

The sensor arrays 27A and 27B are supported by the inner circumferential surface 22a of the travelling gantry 22. The sensor arrays 27A and 27B are disposed to face the diagnostic X-ray sources 26A and 26B with the isocenter C0 interposed therebetween. The sensor arrays 27A and 27B receive the diagnostic X-ray 101 radiated from the diagnostic X-ray sources 26A and 26B and transmitted through a subject near the isocenter C0 and generate a transmission image of the subject. A flat panel detector (FPD), an X-ray image intensifier (II), etc. may, for example, be used as the sensor arrays 27A and 27B.

The radiation therapy device 20 further includes a couch 28 and a couch driving device 29. The couch 28 includes an upper surface 28a on which a patient B being treated by the radiation therapy system 10 lies down.

The couch driving device 29 is controlled by the control device 12 and moves the couch 28. The couch driving device 29 is supported by a base (not illustrated).

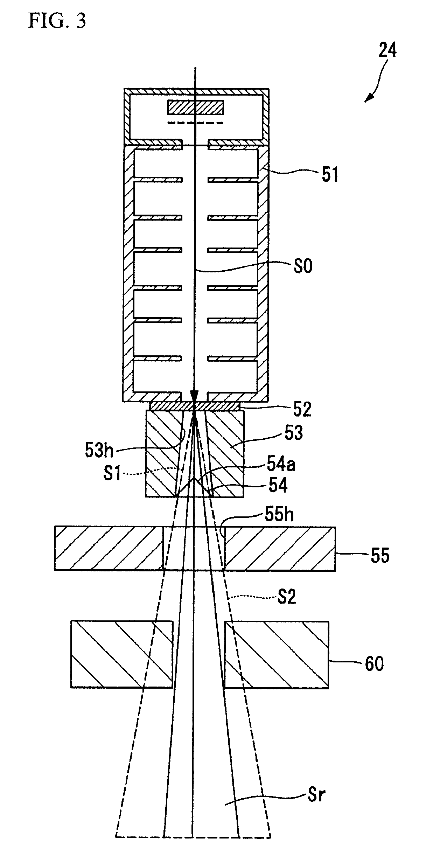

Fig. 3 is a cross-sectional view illustrating the radiation irradiation device 24 which constitutes the radiation therapy device 20.

As illustrated in Fig. 3, the radiation irradiation device 24 includes an electron beam acceleration device 51, an X-ray target 52, a first collimator 53, a flattening filter 54, a second collimator 55, and a multi-leaf collimator 60.

The electron beam acceleration device 51 irradiates the X-ray target 52 with an electron beam S0 generated by accelerating electrons.

The X-ray target 52 is formed of tungsten, a tungsten alloy, etc. The X-ray target 52 emits radiation S1 when irradiated with the electron beam S0.

The first collimator 53 shields some of the radiation S1 so that portions other than desired portions are not irradiated with the radiation S1. The first collimator 53 includes a through-hole 53h though which the radiation S1 radiated from the X-ray target 52 passes. The first collimator 53 is formed of lead, tungsten, etc.

The flattening filter 54 is a filter which substantially evenly distributes dose of the radiation S1 on a plane which is perpendicular to a radiation direction of the radiation S1. The flattening filter 54 is formed of aluminum and the like. The flattening filter 54 is disposed at an outlet side of the through-hole 53h of the first collimator 53. The flattening filter 54 has a protrusion 54a in a substantially conical shape disposed at a side facing the X-ray target 52. The shape of the protrusion 54a is designed so that the dose of the radiation S1 is substantially evenly distributed on the plane which is perpendicular to the radiation direction of the radiation S1.

The second collimator 55 shields some of the radiation S1. The second collimator 55 has a through-hole 55h at a central portion thereof. The second collimator 55 allows radiation S2 to pass therethrough only at the through-hole 55h. The second collimator 55 is formed of lead, tungsten, etc.

By passing through the first collimator 53, the flattening filter 54, and the second collimator 55 described above, some of the radiation S2 having an evenly distributed intensity is further shielded by the multi-leaf collimator 60. The multi-leaf collimator 60 is controlled by the control device 12 and limits a radiation field of the radiation S2. The multi-leaf collimator 60 generates the therapeutic radiation Sr according to properties of radiation which should be radiated to a patient.

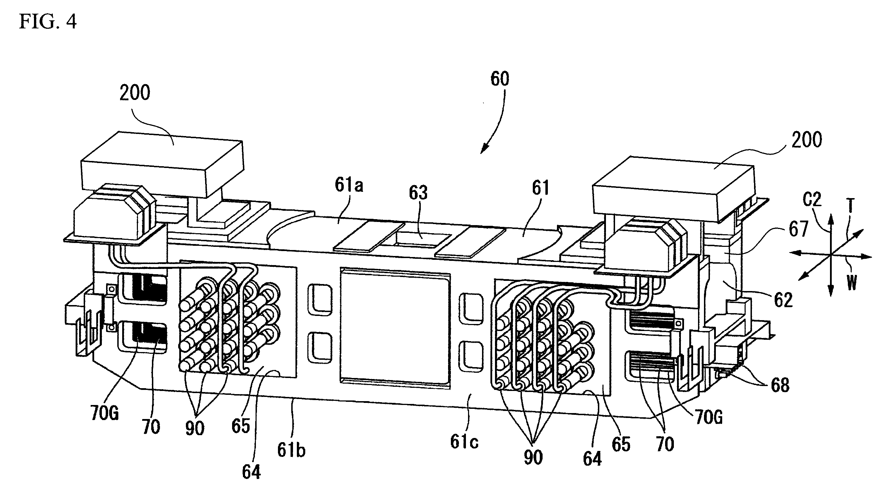

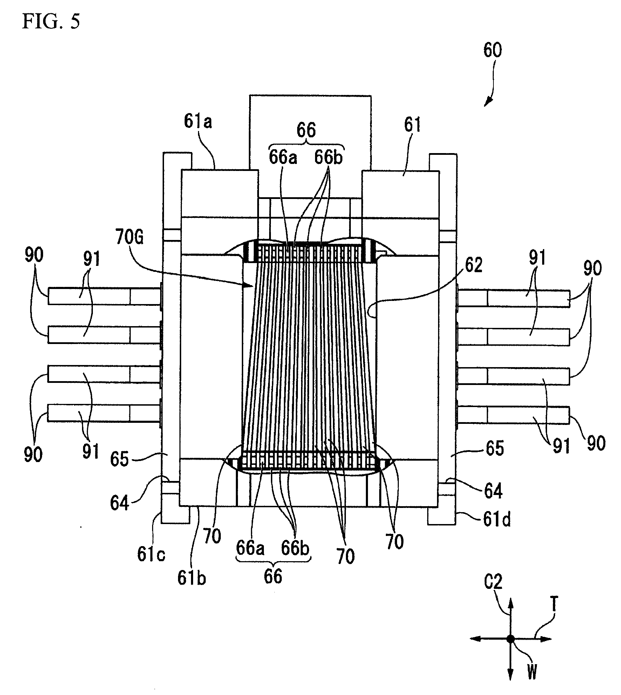

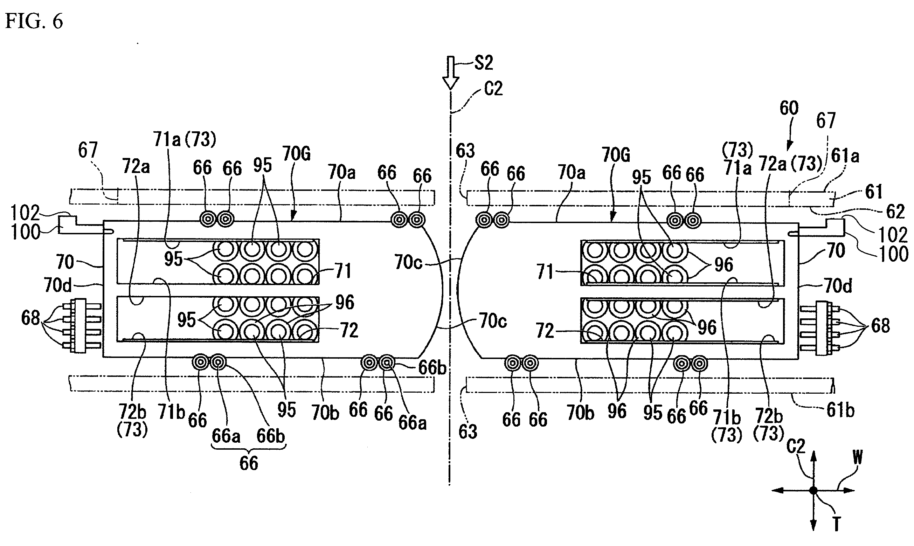

Fig. 4 is a perspective view illustrating an exterior of the multi-leaf collimator 60 which constitutes part of the radiation irradiation device 24. Fig. 5 is a cross-sectional view of the multi-leaf collimator 60 in its width direction. Fig. 6 is a cross-sectional view of the multi-leaf collimator 60 in a direction perpendicular to a second direction which is a thickness direction of a leaf 70 (hereinafter, the second direction will be referred to as plate thickness direction T).

As illustrated in Figs. 4 to 6, the multi-leaf collimator 60 includes a frame 61, a plurality of leaves 70, and a driving device (driving mechanism) 90.

The frame 61 is formed substantially in a rectangular parallelepiped shape which is long in one direction. The frame 61 is disposed such that a first direction which is a longitudinal direction thereof (hereinafter, the first direction will be referred to as a width direction W) is perpendicular to a radiation beam axis of the radiation irradiation device 24. A hollow leaf accommodation unit 62 continuously formed in the width direction W is formed in the frame 61.

In the frame 61, openings 63 which penetrate an outer circumferential side of the frame 61 and the leaf accommodation unit 62 are formed at an upper surface portion 61 a at a side facing the radiation irradiation device 24 and a lower surface portion 61b which is at the opposite side (only the opening 63 at the upper surface portion 61 a is illustrated in Fig. 4). The openings 63 are formed at central portions of the upper surface portion 61a and the lower surface portion 61b in the width direction W.

As illustrated in Figs. 4 and 5, in the frame 61, rectangular openings 64 and 64 are respectively formed at both side surface portions 61 c and 61 d which are perpendicular to the upper surface portion 61a and the lower surface portion 61b. The opening 64 of the side surface portion 61 c and the opening 64 of the side surface portion 61 d are formed to be symmetrical with respect to a virtual plane disposed at the center between the side surface portion 61c and the side surface portion 61 d. Rectangular base plates 65 are mounted on the openings 64.

The leaf 70 is formed in the shape of a substantially rectangular plate. The leaf 70 is formed of, for example, tungsten, a tungsten alloy, etc. through which the radiation S2 is not transmitted.

As illustrated in Fig. 5, a plurality of leaves 70 are arranged at predetermined intervals in the plate thickness direction T. A leaf group 70G is constituted by the plurality of leaves 70. In the embodiment, the leaf group 70G is constituted, for example, by thirty leaves 70. As illustrated in Figs. 4 and 6, a pair of leaf groups 70G are disposed in the leaf accommodation unit 62 in the frame 61 to face each other with a central portion in the width direction W of the frame 61 is interposed therebetween.

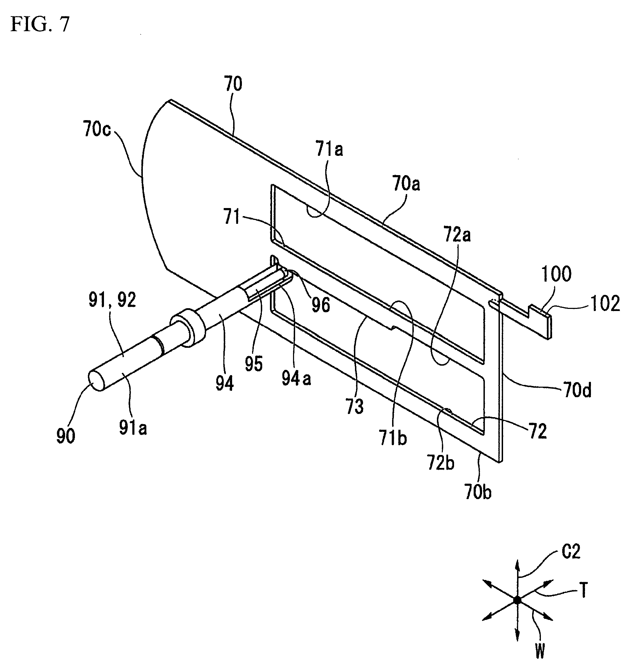

Fig. 7 is a perspective view illustrating the leaf 70 and the driving device 90 which drives the leaf 70.

As illustrated in Figs. 6 and 7, the leaf 70 includes an straight upper edge portion (end surface) 70a and a straight lower edge portion 70b formed parallel to each other. As illustrated in Fig. 6, the upper edge portion 70a is disposed to face the upper surface portion 61a with a gap therebetween in the leaf accommodation unit 62. Likewise, the lower edge portion 70b is disposed to face the lower surface portion 61b with a gap therebetween in the leaf accommodation part 62.

The leaf 70 includes a front edge portion 70c at a side facing the central portion in the width direction W of the frame 61 and formed in the shape of an arc in the leaf accommodation unit 62. In addition, the leaf 70 includes a rear edge portion 70d facing the outside in the width direction W of the frame 61 formed in a straight shape perpendicular to the upper edge portion 70a and a lower edge portion 70b in the leaf accommodation unit 62.

The pair of leaf groups 70G, 70G disposed to face each other with the central portion in the width direction W interposed therebetween in the leaf accommodation unit 62 are disposed so that the front edge portion 70c of each of the leaves faces an area between the opening 63 of the upper surface portion 61a and the opening 63 of the lower surface portion 61b of the frame 61.

Each of the leaves 70 includes slits 71 and 72 that penetrate in the plate thickness direction T. The slits 71 and 72 are continuously formed in a direction in which the front edge portion 70c and the rear edge portion 70d of each of the leaves 70 are connected to each other, i.e., the width direction W. The slits 71 and 72 are arranged at a predetermined interval in a direction in which the upper edge portion 70a and the lower edge portion 70b of the leaf 70 are connected to each other. The slits 71 and 72 are formed closer to the rear edge portion 70d than to the front edge portion 70c to prevent the radiation S2 incident from the opening 63 of the upper surface portion 61a of the frame 61 to an inside of the leaf accommodation unit 62 of the frame 61 from being irradiated thereto.

Each of the leaves 70 includes a rack gear 73 continuously formed in the width direction W on at least one of upper side portions 71a and 72a and lower side portions 71b and 72b of the slits 71 and 72. Here, in the leaf groups 70G of the embodiment, the rack gears 73 of the leaves 70 and 70 adjacent in a direction in which the plurality of leaves 70 are arranged are formed at different sides among the upper side portions 71 a and 72a and the lower side portions 71b and 72b of the slits 71 and 72. In this way, pinion gears 96 which interlock with the rack gears 73 can be prevented from interfering with each other between the leaves 70 and 70 adjacent in the plate thickness direction T.

The plurality of leaves 70 which constitute each of the leaf groups 70G are supported by the frame 61. The frame 61 supports the leaf 70 so that the leaf 70 is capable of advancing and retracting in the width direction W which is perpendicular to the plate thickness direction T. The frame 61 includes a plurality of slide support members 66. The plurality of slide support members 66 are disposed at predetermined intervals in the width direction W at an upper portion and a lower portion of each of the leaf groups 70G. In the embodiment, two slide support members 66 are disposed at each of the central portion side and the outer circumferential side of the frame 61 in the width direction W, i.e., a total of four slide support members 66 which guide movement of the leaf 70 are provided at the upper portion and the lower portion of each of the leaf groups 70G.

As illustrated in Figs. 5 and 6, each of the slide support members 66 includes a shaft 66a fixed to the frame 61 and a plurality of support rollers 66b rotatably mounted on the shaft 66a. The plurality of support rollers 66b are disposed at positions corresponding to the plurality of leaves 70 which constitute the leaf group 70G. The support rollers 66b are constituted to perform a rotation in a direction in which the upper edge portion 70a and the lower edge portion 70b of the leaf 70 extend.

As illustrated in Fig. 6, the support rollers 66b of the slide support members 66 come in contact with the upper edge portion 70a and the lower edge portion 70b of each of the leaves 70. At least two support rollers 66b coming in contact with the upper edge portion 70a and at least two support rollers 66b coming in contact with the lower edge portion 70b are provided.

That is, each of the leaves 70 is supported by the slide support members 66 in the frame 61 in a state in which it can individually advance and retract in the width direction W.

Here, in the leaf group 70G described above, the support rollers 66b of different slide support members 66 among the plurality of slide support members 66 described above may come in contact with the leaves 70 and 70 adjacent in the plate thickness direction T. In this way, the support rollers 66b can be prevented from interfering with each other between the adjacent leaves 70 and 70.

The frame 61 includes a stopper 68 which regulates a movement amount of each of the leaves 70 toward the rear edge portion 70d in the width direction W.

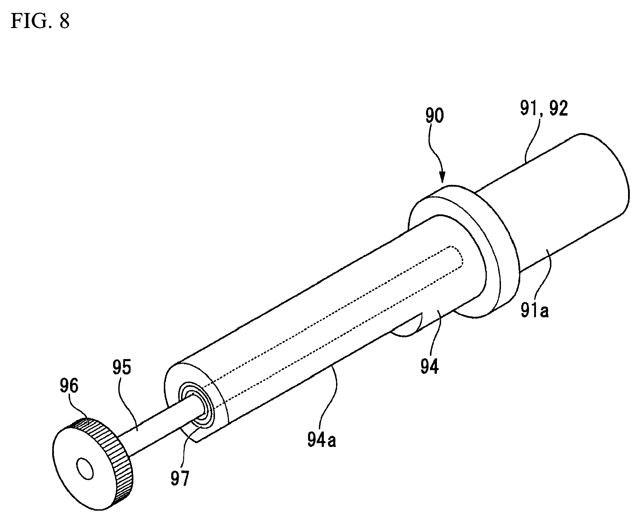

Fig. 8 is a perspective view illustrating a constitution of the driving device 90.

As illustrated in Figs. 7 and 8, the driving device 90 is provided to correspond to each of the plurality of leaves 70. The driving device 90 includes a motor 91, a shaft 95, and the pinion gear 96.

The motor 91 is connected to a proximal end portion of the shaft 95. The motor 91 drives the shaft 95 to rotate about the axis thereof.

Here, as illustrated in Fig. 5, the motor 91 is supported by the base plates 65 provided along the side surface portions 61 c and 61 d of the frame 61.

The motor 91 of the driving device 90, which drives one half of the leaves 70 at a side close to the side surface portion 61c among the plurality of leaves 70 which constitute the leaf group 70G, is supported by the base plate 65 provided at the side surface portion 61 c at one side of the frame 61. The motor 91 of the driving device 90, which drives the other half of the leaves 70 at a side close to the side surface portion 61d among the plurality of leaves 70 which constitute the leaf group 70G, is supported by the base plate 65 provided at the side surface portion 61d at the other side of the frame 61.

As illustrated in Fig. 7, the shaft 95 extends in the plate thickness direction T of the leaf 70. In addition, as illustrated in Figs. 6 and 7, the shaft 95 is inserted into the slit 71 or 72 of the plurality of leaves 70 of the leaf group 70G. In addition, as illustrated in Figs. 7 and 8, the pinion gear 96 is mounted on a distal end portion of the shaft 95. The pinion gear 96 is interlocked with the rack gear 73 formed on any one of the upper side portions 71 a and 72a and the lower side portions 71 b and 72b of the slits 71 and 72 which are a part of the leaf 70.

The driving device 90 further includes a rotary encoder 92 and a cover 94.

The rotary encoder 92 measures a rotational amount of the shaft 95 and outputs the measurement result to the control device 12.

The cover 94 is formed in the shape of a hollow tube. The cover 94 is integrally provided with a housing 91 a of the motor 91. A bearing 97 is provided at an end portion of the cover 94 which is the opposite side from the motor 91. In addition, the shaft 95 is inserted into the cover 94. The shaft 95 is rotatably supported by the bearing 97. That is, the cover 94 supports the shaft 95 by the bearing 97 at a position spaced apart in a direction in which the shaft 95 extends from the motor 91. In this way, the shaft 95 is prevented from being deformed due to a self-weight and the like, and the pinion gear 96 is ensured to be interlocked with teeth of the rack gear 73 even when the motor 91 and the leaf 70 are spaced apart from each other.

A notch 94a is formed at the cover 94 partly in the circumferential direction.

The notch 94a prevents the leaf 70, having the rack gear 73 with which the pinion gear 96 of the shaft 95 inserted into the cover 94 is interlocked, from interfering with another leaf 70 disposed closer to the motor 91.

In the driving device 90, the motor 91 is driven by the control of the control device 12 and rotates the shaft 95. When the shaft 95 rotates, the pinion gear 96 rotates together with the shaft 95, and the rotary force is transmitted to the rack gear 73. Then, the leaf 70 having the rack gear 73 is displaced in the advancing and retracting direction which is the width direction W.

In this way, with respect to each of the pair of leaf groups 70G, when each of the leaves 70 which constitutes the leaf groups 70G is advanced and retracted in the width direction W, some of the radiation S2 incident from the opening 63 of the upper surface portion 61 a of the frame 61 is shielded by the leaf 70 of the leaf groups 70G at both sides. That is, the therapeutic radiation Sr limited to a predetermined shape of a radiation field is generated by the multi-leaf collimator 60.

Treatment is performed as follows in the radiation therapy system 10 described above.

First, a user fixes a patient B to the couch 28 of the radiation therapy device 20 so that the patient B is in a posture indicated by a treatment plan input to the treatment planning device 11.

The control device 12 operates the rotation driving mechanism (not illustrated) and a gantry driving device (not illustrated). Specifically, the control device 12 rotates the ring frame 21 and the travelling gantry 22 about the central axes C1 and C2 and moves the radiation irradiation device 24 so that the therapeutic radiation Sr is irradiated to a position of an affected body part of the patient B at an irradiation angle indicated in the treatment plan. In addition, the control device 12 advances and retracts each of the leaves 70 by the driving device 90 so that the limited shape of the irradiation field of the therapeutic radiation Sr is changed to a shape indicated in the treatment plan input to the treatment planning device 11 in the multi-leaf collimator 60.

Then, the control device 12 uses the radiation irradiation device 24 to irradiate the affected part of the patient B with the therapeutic radiation Sr of a dosage indicated in the treatment plan input to the treatment planning device 11.

As illustrated in Figs. 6 and 7, a marker unit 100 is provided at each of the leaves 70 which constitutes each of the leaf groups 70G of the multi-leaf collimator 60. The marker unit 100 is provided to monitor a position of the leaf 70 advanced and retracted by the driving device 90. The marker unit 100 is formed of a convex portion provided to protrude from the rear edge portion 70d of the leaf 70 in a direction along the upper edge portion 70a.

An identification mark 102 is formed at a whole upper surface of the marker unit 100 to be identifiable at least from a direction perpendicular to both an advancing and retracting direction in which the leaf 70 is advanced and retracted and a thickness direction of the leaf 70, i.e. from an upper portion. The identification mark 102 may be formed with a fluorescent substance. The fluorescent substance may be formed by applying a fluorescent pigment or a fluorescent paint on the upper surface of the marker unit 100 and may also be formed by attaching a fluorescent sheet to the upper surface of the marker unit 100.

As illustrated in Figs. 4 and 6, the marker unit 100 is provided to be exposed upward from an opening 67 formed above the leaf accommodation unit 62 at an upper surface of the frame 61.

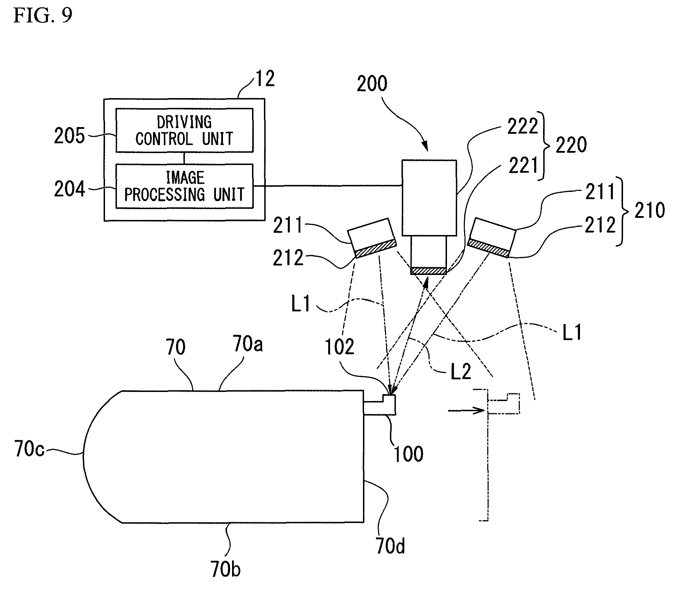

Fig. 9 is a view illustrating a schematic constitution of a mark position detection unit (leaf position detection device) 200 for detecting a position of the identification mark 102 of the marker unit 100 formed on the leaf 70.

As illustrated in Figs. 4 and 9, the radiation therapy device 20 includes the mark position detection unit 200. The mark position detection unit 200 detects a position of the identification mark 102 of the marker unit 100 formed on the leaf 70 in the advancing and retracting direction.

As illustrated in Fig. 9, the mark position detection unit 200 includes an illumination unit 210 and a detection unit 220.

The illumination unit 210 illuminates the identification mark 102 provided at each of the plurality of leaves 70 with detection light L1. The illumination unit 210 includes a light source 211 which emits the detection light L1 and a polarizing plate 212 which polarizes the detection light L1 emitted from the light source 211.

The light source 211 projects light in a visible light range as the detection light L1. For example, a light emitting diode (LED) light source and the like with a high peak of a specific wavelength may be used as the light source 211. Here, a general halogen light source on which a color filter which transmits only light in a wavelength range required for the detection light L1 is mounted may also be used as the light source 211.

The polarizing plate 212 polarizes the detection light L1 in a polarizing direction which is the same as a polarizing direction in a polarizing filter 221 to be described below of the detection unit 220. The polarizing plate 212 of the embodiment blocks unnecessary light not desired to be transmitted through the polarizing filter 221 and does not emit the light as the detection light L1. Here, the polarizing plate 212 may be integrally provided with the light source 211 or may be provided at a position spaced apart from the light source 211.

In the embodiment, the light source 211 and the polarizing plate 212 are disposed above the marker unit 100. In addition, the light source 211 and the polarizing plate 212 are provided in pairs at predetermined intervals in the advancing and retracting direction of the leaf 70. The identification mark 102 may be irradiated with the detection light L1 in a whole region within a stroke range of the advancing and retracting of the leaf 70 by the arrangement of the light source 211 and the polarizing plate 212.

When the detection light L1 is irradiated from the illumination unit 210, the detection unit 220 detects identification light (light) L2 emitted from the identification mark 102. The detection unit 220 includes the polarizing filter 221 and an imaging device 222.

The polarizing filter 221 filters the identification light L2 to be described below reflected from the marker unit 100 and other reflected light reflected from surrounding parts.

The imaging device 222 performs imaging with transmitted light transmitted through the polarizing filter 221.

The identification mark 102 receives the detection light L1 from the illumination unit 210 and emits the identification light L2. The identification light L2 includes reflected light and excited light. Here, the excited light is excited by the fluorescent substance which forms the identification mark 102. The fluorescent substance emits the excited light when irradiated with the detection light L1.

The polarizing filter 221 blocks light other than light in the polarizing direction of the polarizing filter 221 by filtering the identification light L2. In other words, only light matching the polarizing direction of the polarizing filter 221 is transmitted through the polarizing filter 221. The light transmitted through the polarizing filter 221 reaches the imaging device 222 as transmitted light.

The polarizing filter 221 may be integrally provided with the imaging device 222 or may be separately provided by being spaced apart from the imaging device 222.

For example, a charge coupled device (CCD) camera may be used as the imaging device 222. The imaging device 222 is disposed between the pair of the set of the light source 211 and the polarizing plate 212 above the marker unit 100. The imaging device is disposed in such a way that a direction of an optical axis thereof directs downward perpendicularly.

According to the mark position detection unit 200 described above, first, the detection light L1 projected from the light source 211 of the illumination unit 210 is polarized at the polarizing plate 212 in the same polarizing direction as the polarizing filter 221. Afterwards, the identification mark 102 is irradiated with the light polarized by the polarizing plate 212. Then, the identification light L2 which includes reflected light or excited light and reflected light are emitted from the identification mark 102. The identification light L2 and the reflected light emitted from the identification mark 102 are filtered by the polarizing filter 221. In this way, light in a polarizing direction not corresponding to the polarizing direction of the polarizing filter 221 is all blocked. An image of transmitted light transmitted through the polarizing filter 221 is formed on an imaging element (not illustrated) of the imaging device 222. The imaging device 222 records the formed image as image data.

The imaging device 222 is electrically connected to an image processing unit 204. The imaging device 222 outputs the captured image data to the image processing unit 204.

The image processing unit 204 is included as one function of the control device 12 by cooperation between the computer device and a computer program which constitute the control device 12. The image processing unit 204 executes predetermined image processing of the image data output from the imaging device 222. The image processing unit 204 also identifies the identification mark 102 in the image and specifies the position thereof. Here, master data such as a shape, a size, etc. of the identification mark 102 is preregistered in the image processing unit 204 so that the identification mark 102 can be identified.

In addition, an image processing method of the image processing unit 204 is not limited at all, and a known image processing method such as a pattern matching method may be used.

The image processing unit 204 performs identification and position specification with respect to identification marks 102 of all of the leaves 70 included in the image by the image processing. In this way, a position of each of the leaves 70 in the advancing and retracting direction is detected. The image processing unit 204 transmits the detected result to a driving control unit 205 of the driving device 90 in the control device 12.

The driving control unit 205 executes feedback control of the driving device 90 based on the position of each of the leaves 70 and compensates a control value of the motor 91 as needed.

Consequently, according to the mark position detection unit 200, the multi-leaf collimator 60, and the radiation therapy device 20 described above, after the identification light L2 is filtered by the polarizing filter 221, an image of the identification light L2 is formed by the imaging device 222. In this way, when light in a wavelength range longer than that of an ultraviolet light source (visible light range) is used as the detection light L1, some of the excited light and the reflected light of the identification light L2 is restrained by the polarizing filter 221 even when intense detection light L1 is emitted to obtain sufficient excited light.

As a result, identification of a position of the leaf 70 can be ensured even when the detection light L1 in the visible light range is used as the light source 211. In addition, an expensive ultraviolet light source is not required to be used, and handling of the radiation therapy device 20 is facilitated.

In addition, unnecessary light not transmitted through the polarizing filter 221 can be blocked in advance by the polarizing plate 212 provided at the illumination unit 210. In this way, reflected light unnecessary for the identification mark 102 can be suppressed in advance and identifiability of the identification mark 102 can be improved.

In addition, the present invention is not limited to the embodiment described above and includes various modifications to the embodiment described above within the scope not departing from the gist of the present invention. That is, specific shapes or constitutions, etc. mentioned in the embodiment are merely examples and may be properly modified.

### (First modified example)

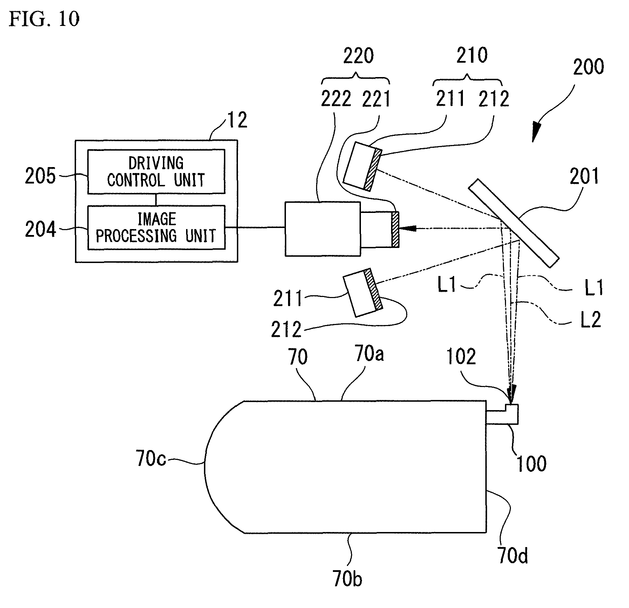

Fig. 10 is a view illustrating a constitution of the mark position detection unit 200 according to a first modified example of the embodiment.

As illustrated in Fig. 10, the mark position detection unit 200 according to the first modified example includes a mirror 201. The mirror 201 is disposed above rear end portions of the plurality of leaves 70 which constitute the leaf group 70G. The illumination unit 210 and the detection unit 220 are disposed above the frame 61 illustrated in Fig. 4.

Optical axes of the imaging device 222 and the polarizing filter 221 which constitute the detection unit 220 are provided to be parallel to the advancing and retracting direction of the leaf 70. The light source 211 and the polarizing plate 212 which constitute the illumination unit 210 are disposed above and below the imaging device 222.

The illumination unit 210 and the detection unit 220 perform emission of the detection light L1 and image formation of the identification light L2 through the mirror 201.

The light source 211 of the illumination unit 210 emits the detection light L1 transmitted through the polarizing plate 212 to the rear end portions of the plurality of leaves 70 through the mirror 201. In this way, the identification light L2 is emitted from the identification mark 102 of the rear end portion of each of the plurality of leaves 70. The imaging device 222 of the detection unit 220 forms an image of the identification light L2 through the mirror 201 and the polarizing filter 221 to obtain the image data.

Consequently, according to the constitution of the first modified example, a degree of freedom of a layout of the illumination unit 210 and the detection unit 220 can be improved by using the mirror 201. In addition, in the constitution of the first modified example, since the optical axes of the imaging device 222 and the polarizing filter 221 are provided to be parallel to the advancing and retracting direction of the leaf 70, the height of the mark position detection unit 200 can be suppressed.

In addition, as in the forementioned embodiment, since some of the excited light and the reflected light of the identification light L2 is restrained by the polarizing filter 221 in the constitution of the first modified example, imaging by the imaging device 222 can be performed without causing halation. In this way, a position of the leaf 70 can be identified even when the detection light L1 in the visible light range is used as the light source 211. As a result, an expensive ultraviolet light source is not required to be used, and handling of the radiation therapy device 20 is facilitated.

In addition, although the light source 211 and the polarizing plate 212 which constitute the illumination unit 210 are disposed above and below the imaging device 222 in the first modified example, embodiments are not limited thereto, and the light source 211 and the polarizing plate 212 may also be provided at both sides with the imaging device 222 interposed therebetween in the plate thickness direction of the leaf 70.

### (Second modified example)

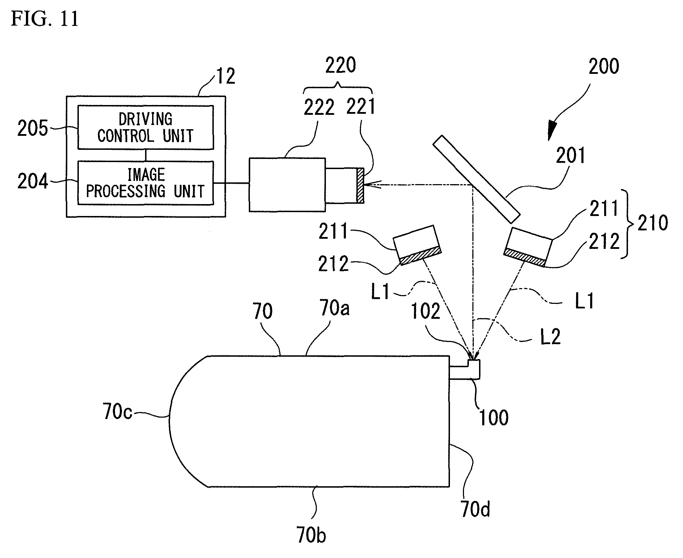

Fig. 11 is a view illustrating a constitution of the mark position detection unit 200 according to a second modified example of the embodiment.

As illustrated in Fig. 11, as in the first modified example, the mark position detection unit 200 according to the second modified example includes the mirror 201. The optical axes of the imaging device 222 and the polarizing filter 221 which constitute the detection unit 220 are provided to be parallel with the advancing and retracting direction of the leaf 70.

The light source 211 and the polarizing plate 212 which constitute the illumination unit 210 are disposed at both sides with a position vertically above the identification mark 102 interposed therebetween in the advancing and retracting direction of the leaf 70.

Here, the arrangement of the detection unit 220 described above is not limited to that illustrated in Fig. 11. For example, the detection unit 220 may be disposed at a position symmetrical to that of the leaf 70 with the position vertically above the identification mark 102 interposed therebetween, i.e., at the opposite side of the leaf 70 with the position vertically above the identification mark 102 interposed therebetween.

In the mark position detection unit 200, the light source 211 of the illumination unit 210 emits the detection light L1 transmitted through the polarizing plate 212 to the rear end portions of the plurality of leaves 70. The imaging device 222 of the detection unit 220 images the identification mark 102 at the rear end portion of each of the plurality of leaves 70 through the mirror 201 and the polarizing filter 221.

According to the constitution of the second modified example, a degree of freedom of a layout of the detection unit 220 can be improved by using the mirror 201. In addition, according to the constitution of the second modified example, the height of the mark position detection unit 200 can be suppressed since the optical axes of the imaging device 222 and the polarizing filter 221 are provided to be parallel to the advancing and retracting direction of the leaf 70.

In addition, as in the forementioned embodiment, according to the constitution of the second modified example, since some of the excited light and the reflected light of the identification light L2 is restrained by the polarizing filter 221 in the constitution of the second modified example, imaging by the imaging device 222 can be performed without causing halation. In this way, a position of the leaf 70 can be identified even when the detection light L1 in the visible light range is used as the light source 211. As a result, an expensive ultraviolet light source is not required to be used, and handling of the radiation therapy device 20 is facilitated.

Here, in the constitution of the second modified example, the light source 211 and the polarizing plate 212 which constitute the illumination unit 210 are disposed at both sides with the position vertically above the identification mark 102 interposed therebetween in the advancing and retracting direction of the leaf 70. However, embodiments are not limited to this constitution, and, for example, the light source 211 and the polarizing plate 212 may also be disposed at both sides with the position vertically above the identification mark 102 interposed therebetween in the plate thickness direction of the leaf 70.

In addition, in the second modified example, the detection unit 220 performs imaging through the mirror 201, and the illumination unit 210 directly emits the detection light L1 to the rear end portion of the leaf 70. However, embodiments are not limited to this constitution. The arrangement of the detection unit 220 and the illumination unit 210 of the second modified example may be changed such that the illumination unit 210 illuminates through the mirror 201 and the detection unit 220 directly images the identification mark 102.

Although the mirror 201 is used in the constitutions of the first modified example and the second modified example, the mirror 201 may be installed at any angle, and in any number and layout as long as the irradiation of the detection light L1 and the imaging of the identification light L2 from the identification mark 102 can be performed.

In addition, although the light sources 211 are provided in a pair at a predetermined interval in the advancing and retracting direction of the leaf 70, embodiments are not limited thereto. There may also be only one light source 211, and the light source 211 may be, for example, in a ring shape.

In addition, the constitution may not include the polarizing plate 212.

In addition, although the marker unit 100 and the mark position detection unit 200 are provided at the rear edge portion 70d of the leaf 70 in the embodiment, the first modified example, and the second modified example, the marker unit 100 and the mark position detection unit 200 may also be provided at a side of the front edge portion 70c. In addition, although the identification mark 102 is formed of a fluorescent substance, embodiments are not limited to the fluorescent substance, and, for example, the identification mark 102 may also be formed by applying white paint, pigment, or a seal, etc.

### [Industrial Applicability]

Identification of an identification mark provided on a leaf can be ensured by filtering light emitted from an identification mark when the identification mark is illuminated with detection light with a polarizing filter and detecting the transmitted light.

### [Reference Signs List]

- 10: Radiation therapy system
- 11: Treatment planning device
- 12: Control device (control unit)
- 20: Radiation therapy device
- 21: Ring frame
- 21a: Lower end portion
- 22: Travelling gantry
- 22a: Inner circumferential surface
- 23: Sensor array
- 24: Radiation irradiation device
- 25: Rotation shaft
- 26A, 26B: X-ray source
- 27A, 27B: Sensor array
- 28: Couch
- 28a: Upper surface
- 29: Couch driving device
- 51: Electron beam acceleration device
- 52: X-ray target
- 53: First collimator
- 53h: Through-hole
- 54: Flattening filter
- 54a: Protrusion
- 55: Second collimator
- 55h: Through-hole
- 60: Multi-leaf collimator
- 61: Frame
- 61a: Upper surface portion
- 61b: Lower surface portion
- 61c, 61d: Side surface portion
- 62: Leaf accommodation unit
- 64: Opening
- 65: Base plate
- 66: Slide support member
- 66a: Shaft
- 66b: Support roller
- 67: Opening
- 68: Stopper
- 70: Leaf
- 70G: Leaf group
- 70a: Upper edge portion
- 70b: Lower edge portion
- 70c: Front edge portion
- 70d: Rear edge portion
- 71, 72: Slit
- 71a, 72a: Upper side portion
- 71b: Lower side portion
- 73: Rack gear
- 90: Driving device (driving mechanism)
- 91: Motor
- 91a: Housing
- 92: Rotary encoder
- 94: Cover
- 95: Shaft
- 96: Pinion gear
- 97: Bearing
- 100: Marker unit
- 101: Diagnostic X-rays
- 102: Identification mark
- 200: Mark position detection unit (leaf position detection device)
- 201: Mirror
- 204: Image processing unit
- 205: Driving control unit
- 210: Illumination unit
- 211: Light source
- 212: Polarizing plate
- 220: Detection unit
- 221: Polarizing filter
- 222: Imaging device
- B: Patient
- C0: Isocenter
- C1: Central axis
- C2: Central axis
- L1: Detection light
- L2: Identification light (light)
- S0: Electron beam
- S1: Radiation
- S2: Radiation
- Sr: Therapeutic radiation

## Claims

**1.** A leaf position detection device which detects a position of a leaf of a multi-leaf collimator having a plurality of the leaves which limit a radiation field of radiation, the leaf position detection device comprising:
an illumination unit configured to illuminate an identification mark provided on the leaf with detection light; and
a detection unit configured to detect a light emitted from the identification mark illuminated with the detection light,
wherein the illumination unit includes a light source configured to emit the detection light, and
wherein the detection unit includes a polarizing filter configured to filter the light emitted from the identification mark and an imaging device configured to detect a transmitted light filtered by the polarizing filter.

**2.** The leaf position detection device according to claim 1, wherein the identification mark is composed of a fluorescent substance.

**3.** The leaf position detection device according to claims 1 or 2, wherein the illumination unit further includes a polarizing plate configured to polarize the detection light radiated from the light source.

**4.** A multi-leaf collimator which limits a radiation field of radiation, the multi-leaf collimator comprising:
a plurality of leaves arranged in a thickness direction of the leaf;
a driving mechanism configured to advance and retract each of the leaves with respect to the radiation;
an identification mark provided on each of the leaves and identifiable at least from a direction perpendicular to both the advancing and retracting direction in which the leaf is advanced and retracted and the thickness direction of the leaf; and
the leaf position detection device according to any one of claims 1 to 3.

**5.** A radiation therapy device comprising:
the multi-leaf collimator according to claim 4;
a control unit configured to control the driving mechanism based on a position of the identification mark detected by the leaf position detection device; and
a radiation irradiation device configured to radiate radiation.

**1.** A leaf position detection device which detects a position of a leaf of a multi-leaf collimator having a plurality of leaves which limit a radiation field of radiation, the leaf position detection device comprising:
an illumination unit configured to illuminate an identification mark composed of a fluorescent substance provided on the leaf with detection light in a visible light range; and
a detection unit configured to detect light emitted from the identification mark which is illuminated with the detection light,
wherein the illumination unit includes a light source configured to emit the detection light, and
wherein the detection unit includes a polarizing filter configured to filter the light emitted from the identification mark and an imaging device configured to detect a transmitted light filtered by the polarizing filter.

**2.** The leaf position detection device according to claim 1, wherein the illumination unit further includes a polarizing plate configured to polarize the detection light emitted from the light source.

**3.** A multi-leaf collimator which limits a radiation field of radiation, the multi-leaf collimator comprising:
a plurality of leaves arranged in a thickness direction of the leaf;
a driving mechanism configured to advance and retract each of the leaves with respect to the radiation;
an identification mark provided on each of the leaves and identifiable at least from a direction perpendicular to both the advancing and retracting direction in which the leaf is advanced and retracted and the thickness direction of the leaf; and
the leaf position detection device according to claim 1 or 2.

**4.** A radiation therapy device comprising:
the multi-leaf collimator according to claim 3;
a control unit configured to control the driving mechanism based on a position of the identification mark detected by the leaf position detection device; and
a radiation irradiation device configured to radiate radiation.

## Amended claims

### Amended claims under Art. 19.1 PCT

**1.** Currently Amended) A leaf position detection device which detects a position of a leaf of a multi-leaf collimator having a plurality of leaves which limit a radiation field of radiation, the leaf position detection device comprising:
an illumination unit configured to illuminate an identification mark composed of a fluorescent substance provided on the leaf with detection light in a visible light range; and
a detection unit configured to detect light emitted from the identification mark which is illuminated with the detection light,
wherein the illumination unit includes a light source configured to emit the detection light, and
wherein the detection unit includes a polarizing filter configured to filter the light emitted from the identification mark and an imaging device configured to detect a transmitted light filtered by the polarizing filter.

**2.** Cancelled)

**3.** Currently Amended) The leaf position detection device according to claim 1, wherein the illumination unit further includes a polarizing plate configured to polarize the detection light emitted from the light source.

**4.** Currently Amended) A multi-leaf collimator which limits a radiation field of radiation, the multi-leaf collimator comprising:
a plurality of leaves arranged in a thickness direction of the leaf;
a driving mechanism configured to advance and retract each of the leaves with respect to the radiation;
an identification mark provided on each of the leaves and identifiable at least from a direction perpendicular to both the advancing and retracting direction in which the leaf is advanced and retracted and the thickness direction of the leaf; and
the leaf position detection device according to claim 1 or 3.

**5.** Currently Amended) A radiation therapy device comprising:
the multi-leaf collimator according to claim 3;
a control unit configured to control the driving mechanism based on a position of the identification mark detected by the leaf position detection device; and
a radiation irradiation device configured to radiate radiation.

Statement under Art. 19.1 PCT
The description "composed of a fluorescent substance" is added to claim 1 of the claims. The amendment is content that was described in claim 2 of the claims which is cancelled and is based on paragraphs [0061] and [0067] of the specification. "Identification mark" in claim 1 of the claims is clarified by the amendment. In addition, the description "in a visible light range" is added to claim 1 of the claims. The amendment is based on paragraphs [0064], [0074], and [0080] of the specification. "Detection light" in claim 1 is clarified by the amendment.

Dependent claims 3, 4, and 5 of the claims are changed according to the cancellation of claim 2.

According to the present invention, in the case of performing image processing, even when image processing is not performed in a stable environment in which a cover is installed and a disturbance light emitted from a surrounding illumination is shielded, stable image processing can be performed even when surroundings are bright. In addition, identification of a position of a leaf can be more ensured even when detection light in a visible light range is used as a light source. In addition, an expensive ultraviolet light source is not required to be used, and handling of the radiation therapy device is facilitated.
